## Europäisches Patentamt
## European Patent Office
## Office européen des brevets

(19)

(11) Publication number: **0 323 865**
A1

(12)

# EUROPEAN PATENT APPLICATION

(21) Application number: **89200003.5**

(22) Date of filing: **02.01.89**

(51) Int. Cl.⁴: **C 12 P 1/06**
A 61 K 35/66, A 61 K 39/00
//(C12P1/06,C12R1:55)

(30) Priority: **07.01.88 US 142620**

(43) Date of publication of application:
**12.07.89 Bulletin 89/28**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900 (US)**

(72) Inventor: **Monaghan, Richard L.**
**48 Johnson Road**
**Somerset New Jersey 08873 (US)**

**Sigal, Nolan H.**
**722 Shackamaxon Drive**
**Westfield New Jersey 07090 (US)**

**Kaplan, Louis**
**7 Lexington Road**
**Rockland County New City New York 10956 (US)**

**Byrne, Keven M.**
**6 Forest Lane**
**West Trenton New Jersey 08628 (US)**

**Borris, Robert P.**
**134 Fox Hollow Road**
**Glen Gardner New Jersey 08826 (US)**

**Dumont, Francis J.**
**1171 Main Street Apt. 1F**
**Rahway New Jersey 07065 (US)**

**Garrity, George M.**
**617 Clark Street Westfield**
**New Jersey 07090 (US)**

**Zink, Deborah L.**
**37 Blenheim Road Manalapan**
**New Jersey 07726 (US)**

(74) Representative: **Hesketh, Alan, Dr.**
**European Patent Department Merck & Co., Inc. Terlings**
**Park Eastwick Road**
**Harlow Essex, CM20 2QR (GB)**

(54) **Novel immunosuppressant agent.**

(57) Described is a process for producing a new immunosup-
pressant, "immunomycin", by novel fermentation conditions of
Streptomyces hygroscopicus var. ascomyceticus. The immuno-
suppressant is useful in preventing human host rejection of
foreign organ transplants, e.g. bone marrow and heart
transplants. Also described is a new method of use utilizing
known "ascomycin" as an immunosuppressant.

**Description**

## NOVEL IMMUNOSUPPRESSANT AGENT

### BACKGROUND OF THE INVENTION

#### 1. Field of the Invention

This invention relates to a new immunosuppressant agent, "immunomycin", a novel process for its production, as well as a new method of use for "ascomycin" as an immunosuppressant, and a method of their use in a human host for treatment of autoimmune diseases, infectious diseases and/or prevention of organ transplant rejections.

#### 2. Brief Description of Disclosures in the Art

In 1983, the US FDA licensed cyclosporin, an extremely effective anti-rejection drug that revolutionized the field of organ transplant surgery. The drug acts by inhibiting the body's immune system from mobilizing its vast arsenal of natural protecting agents to reject the transplant's foreign protein.

As effective as the drugs are in fighting transplantation rejection, they suffer drawbacks in causing kidney failure, liver damage. and ulcers which in many cases can be very severe.

Newer, safer drugs exhibiting less side effects are constantly being searched for in the field.

EPO Publication No. 0184162 to Fujisawa, hereby incorporated by reference, describes a new macrolide immunosuppressant FK-506 which is reputed to be 100 times more effective than cyclosporin. The macrolide is produced by fermentation of a particular strain of Streptomyces tsukubaensis. Also described is the closely related macrolide immunosuppressant FK-520, produced by S. hygroscopicus subsp. yakushimaensis.

USP 3,244,592 to T. Arai describes the culturing of Streptomyces hygroscopicus var. ascomyceticus to produce the antifungal "ascomycin". There is, however, no description of the production of any materials which are immunosuppressive.

### SUMMARY OF THE INVENTION

It has been found that a new immunosuppressant, "immunomycin", can be obtained by fermentating Streptomyces hygroscopicus var. ascomyceticus under submerged aerobic conditions in an aqueous carbohydrate medium, containing a nitrogen nutrient, said conditons being conducted at a pH of less than 8.0.

The resultant "immunomycin" exhibits immunosuppressive activity, i.e., positive inhibition of T-cell activation, as demonstrated by the calcium ionophore (ionomycin) phorbol myristate acetate (PMA) induced T-cell stimulation assay, also referred to herein as the "T-cell proliferation assay".

The principle of this assay is to measure the proliferation of mouse T lymphocytes stimulated with the combination of ionomycin plus PMA. A positive sample in this assay will inhibit T-cell proliferation, as indicated by reduced tritiated thymidine uptake.

The antifungal, ascomycin, as described in USP 3,244,592, hereby incorporated by reference for this purpose, also exhibits immunosuppressive activity.

In accordance with this invention, there is provided: a process for producing an immunosuppressant, identified as "immunomycin", comprising the step of culturing a strain of Streptomyces hygroscopicus var. ascomyceticus under submerged aerobic fermentation conditions at a pH below 8.0, in an aqueous carbohydrate medium, containing a nitrogen nutrient, for a sufficient time to produce product "immunomycin".

Further provided is a new immunosuppressant, "immunomycin", produced by the above process which exhibits: positive inhibition of T-cell activation by the T-cell proliferation assay and a molecular weight as determined by mass spectroscopy of 791.4784.

Also provided is a pharmaceutical composition containing a therapeutically effective amount of "immunomycin" in combination with a pharmaceutically acceptable, substantially non-toxic carrier or excipient.

In addition, there is provided a method of use for treating a human host to prevent transplantation rejection, or for treating autoimmune disease or infectious disease comprising administering to said host a therapeutically effective amount of "immunomycin".

Also provided is a method of use for treating a human host to prevent transplantation rejection, or for treating autoimmune disease or infectious disease comprising administering to said host a therapeutically effective amount of "ascomycin".

### DETAILED DESCRIPTION OF THE INVENTION AND PREFERRED EMBODIMENTS

The present invention involves the fermentation of Streptomyces hygroscopicus var. ascomyceticus a microorganism which is extensively described in USP 3,244,592 with respect to its physical characteristics and taxonomy and is hereby incorporated by reference for this particular purpose.

The present invention can be practiced with any "immunomycin-producing" strain of S. hygroscopicus var. ascomyceticus and particularly preferred is the ATCC 14891 strain.

In general, "immunomycin" can be produced by culturing (fermenting) the immunomycin substance-producing strain in an aqueous nutrient medium containing sources of assimilable carbon and nitrogen, preferably under submerged aerobic conditions (e.g. shaking culture, submerged culture, etc.). The aqueous medium must be maintained below pH 8.0 at the initiation and termination (harvest) of the fermentation process. A higher pH leads to substantial and/or total loss of product. The desired pH may be maintained by the use of a buffer such as morpholinoethanesulfonic acid (MES), morpholinopropanesulfonic acid (MOPS), and the like, or by choice of nutrient materials which inherently possess buffering properties, e.g. KQ and FKSH production media described herein below.

The preferred sources of carbon in the nutrient medium are carbohydrates such as glucose, xylose, galactose, glycerin, starch, dextrin, and the like. Other sources which may be included are maltose, rhamnose, raffinose, arabinose, mannose, salicin, sodium succinate, and the like.

The preferred sources of nitrogen are yeast extract, meat extract, peptone, gluten meal, cottonseed meal, soybean meal and other vegetable meals (partially or totally defatted), casein hydrolysates, soybean hydrolysates and yeast hydrolysates, corn steep liquor, dried yeast, wheat germ, feather meal, peanut powder, distilled solubles, etc., as well as inorganic and organic nitrogen compounds such as ammonium salts (e.g. ammonium nitrate, ammonium sulfate, ammonium phosphate, etc.), urea, amino acids, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, magnesium salts, copper salts, cobalt salts, and the like. If necessary, especially when the culture medium foams seriously, a defoaming agent, such as liquid paraffin, fatty oil, plant oil, mineral oil or silicone may be added.

As the conditions for the production of immunomycin in massive amounts, submerged aerobic cultural conditions are preferred therefor. For the production in small amounts, a shaking or surface culture in a flask or bottle is employed. Furthermore, when the growth is carried out in large tanks, it is preferable to use the vegetative form of the organism for inoculation in the production tanks in order to avoid growth lag in the process of production of immunomycin. Accordingly, it is desirable first to produce a vegetative inoculum of the organism by inoculating a relatively small quantity of culture medium with spores or mycelia of the organism produced in a "slant" and culturing said inoculated medium, also called the "seed medium", and then to transfer the cultured vegetative inoculum aseptically to large tanks. The fermentation medium, in which the vegetative inoculum is produced, is substantially the same as or different from the medium utilized for the production of immunomycin and is generally autoclaved to sterilize the medium prior to inoculation. The pH of the medium is generally adjusted to below 8.0 prior to the autoclaving step by suitable addition of an acid or base, preferably in the form of a buffering solution.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

The fermentation is usually conducted at a temperature between about 20°C and 40°C, preferably 25-35°C, for a period of about 50 hours to 150 hours, which may be varied according to fermentation conditions and scales. Preferably, the production cultures are incubated for about 96 hours at 27°C on a rotary shaker operating at 220 rpm, wherein the pH of the fermentation medium is maintained at below 8.0 to harvest.

Preferred culturing/production media for carrying out the fermentation include the following media:

KO: comprising figs, dextrin, primary yeast, $COCl_2 \bullet 6H_2O$ and an antifoam agent such as P-2000 (Dow Corning), in preferred weight percentages of the fermentation medium respectively as: 3.0%, 1.5%, 1.0%, 0.005% and 0.25% (volume/volume). The pH of this medium is generally adjusted to about 7.4 prior to autoclaving and inoculation by the seed culture.

RP21: comprising glucose, glycerol, ammonium sulfate, potassium dihydrogen phosphate, potassium monohydrogen phosphate, L-lysine, nutrisoy, and a buffer such as morpholinoethanesulfonic acid (MES), in preferred weight percentages of the fermentation medium respectively as: 2.0%, 2.0%, 0.5%, 0.25%, 0.25%, 0.4%, 3.0%, and 2.13%. The pH of this medium is generally adjusted to about 6.3 prior to autoclaving and inoculation by the seed culture.

FKSH: comprising glucose, corn steep liquor, primary yeast, corn gluten meal, wheat germ, and $CaCO_3$, in preferred weight percentages of the fermentation medium respectively as: 4.5%, 1.0%, 1.0%, 1.0%, 0.5%, and 0.1%. The pH of the medium is generally adjusted to about 6.8 prior to autoclaving and inoculating with the seed culture.

Preferred production media described above are the RP21 and FKSH, with the FKSH medium yielding slightly higher yields, and the RP21 medium being more convenient to work with, possessing a more fluid consistency.

The produced immunomycin can be recovered from the culture medium by conventional means which are commonly used for the recovery of other known biologically active substances. The immunomycin substance produced is found in the cultured mycelium and filtrate, and accordingly can be isolated and purified from the mycelium and the filtrate, which are obtained by filtering or centrifuging the cultured broth, by a conventional method such as concentration under reduced pressure, lyophilization, extraction with a conventional solvent, such as methanol and the like, pH adjustment, treatment with a conventional resin (e.g. anion or cation exchange resin, non-ionic adsorption resin, etc.), treatment with a conventional adsorbent (e.g. activated charcoal, silicic acid, silica gel, cellulose, alumina, etc.), crystallization, recrystallization, and the like. A preferred method is solvent extraction, particularly using methanol.

The broth from the fermentation exhibits positive immunosuppressive activity by the "T-cell proliferation assay" and possesses utility on this basis.

The product "immunomycin" exhibits the following physical characteristics:

1. White amorphous powder

2. Solubility in methanol

3. Molecular weight determined by mass spectroscopy of 791.4784, consistent with an empirical formula of $C_{43}H_{69}NO_{12}$

The immunomycin obtained according to the fermentation processes as explained above can be isolated and purified in a conventional manner, for example, extraction, precipitation, fractional crystallization, recrystallization, chromatography, and the like.

Suitable salts of the material may include pharmaceutically acceptable salts such as basic salts, for example, alkali metal salt (e.g. sodium salt, potassium salt, etc.), alkaline earth metal salt (e.g. calcium salt, magnesium salt, etc.), ammonium salt, amine salt (e.g. triethylamine salt, N-benzyl-N-methylamine salt, etc.) and other conventional organic salts.

It is to be noted that in the aforementioned fermentation reactions and the post-treatment of the fermentation mixture therein, the conformer and/or stereo isomer(s) of immunomycin due to asymmetric carbon atom(s) or double bond(s) of the immunomycin may occasionally be transformed into the other conformer and/or stereoisomer(s), and such cases are also included within the scope of the present invention.

The antifungal "ascomycin", described in USP 3,244,592, hereby incorporated by reference, possesses immunosuppressive activity and thus is also useful as an immunosuppressant, as described also herein for "immunomycin".

The immunomycin of the present invention, as well as ascomycin, possesses pharmacological activity such as immunosuppressive activity, antimicrobial activity, and the like, and therefore are useful for the treatment and prevention of the transplantation rejection of organs or tissues such as heart, kidney, liver, medulla ossium, skin, etc., graft-versus-host diseases by medulla ossium transplantation, autoimmune diseases such as rheumatoid arthritis, systemic lupus erythematosus, Hashimoto's thyroiditis, multiple sclerosis, myasthenia gravis, type I diabetes, uveitis, etc., infectious diseases caused by pathogenic microorganisms, and the like.

The pharmaceutical composition of this invention can be used in the form of a pharmaceutical preparation, for example, in solid, semisolid or liquid form, which contains the immunomycin, or ascomycin, of the present invention, as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of diseases.

For applying this composition to a human, it is preferable to apply if by parenteral or enteral administration. While the dosage of therapeutically effective amount of the immunomycin, or ascomycin, varies from, and also depends upon the age and condition of each individual patient to be treated, a daily dose (calculated on the basis of a 70 kg man) of about 0.01-1000 mg, preferably 0.1-500 mg and more preferably 0.5-100 mg, of the active ingredient is generally given for treating diseases, and an average single dose of about 0.5 mg, 1 mg, 5 mg, 10 mg, 50 mg, 100 mg, 250 mg and 500 mg is generally administered.

The following examples are given for the purpose of illustrating the present invention and should not be construed as being limitations on the scope or spirit of the instant invention.

## EXAMPLE 1

### Microorganism and Culture Conditions

Streptomyces hygroscopicus var. ascomyceticus was obtained from the American Type Culture Collection (ATCC No. 14891). The lyophilized culture was suspended in 2 ml saline, and 0.2 ml used to inoculate an ISP4-NZE slant (ISP Medium 4, Difco Laboratories, supplemented with 0.066% N-Z Amine E, Sheffield Products). Slants were incubated for 2 to 3 days at 27°C. The surface of a slant was scraped and used to inoculate a 250 ml baffled shake flask containing 50 ml of an autoclaved (sterilized) seed medium consisting of dextrin 1.0%, glucose 0.1%, beef extract 0.3%, ardamine PH (Yeast Products, Inc.) 0.5%, N-Z Amine type E 0.5%, $MgSO_4.7H_2O$ 0.005%, $KH_2PO_4$ 0.0091%, $Na_2HPO_4$ 0.0095%, and $CaCO_3$ 0.05%. The PH of the seed medium was adjusted to 7.1 before autoclaving. The seed was incubated in the seed medium at 27°C for 24 hours on a rotary shaker operating at 220 rpm. A 1.5 ml aliquot of the resulting seed medium was used to inoculate a 250 ml non-baffled shake flask containing 45 ml of each of the following three different previously autoclaved (sterilized) production media, which were subsequently incubated for 96 hours at 27°C on a rotary shaker operating at 220 rpm:

1. KO production medium consisting of figs 3.0%, dextrin 1.5%, primary yeast 1.0%, $CoCl_2 .6H_2O$ 0.001%, antifoam P-2000 (Dow Corning) 0.25% (V/V), where the pH was adjusted to 7.4 before autoclaving, and which yielded a broth containing a 20 microgram/ml titer of immunomycin.

2. RP21 production medium consisting of glucose 2.0%, glycerol 2.0%, $(NH_4)_2SO_4$ 0.5%, $KH_2PO_4$ 0.25%, $K_2HPO_4$ 0.25%, L-lysine 0.4%, nutrisoy 3.0%, morpholinoethanesulfonic acid (MES) buffer 2.13%, where the pH was adjusted to 6.3 before autoclaving, and which yielded a broth containing a 20 microgram/ml titer of immunomycin.

3. FKSH production medium consisting of glucose 4.5%, corn steep liquor 1.0%, primary yeast 1.0%, corn gluten meal 1.0%, wheat germ 0.5%, $CaCO_3$ 0.1%, where the pH was adjusted to 6.8 before autoclaving, and which yielded a broth containing a 40 microgram/ml titer of immunomycin.

## EXAMPLE 2

### T-Cell Proliferation Assay on the above Broths

#### 1. Sample Preparation

An equal volume of methanol was added to a 1 ml sample of each of the above broths producing a 1:2 whole broth equivalent (WBE). The samples were centrifuged at 3,000 rpm for 5 minutes and the resulting supernatants were used as starting materials for the T-cell proliferation assay.

#### 2. Assay

Spleens from C57Bl/6 mice were taken under sterile conditions and gently dissociated in ice-cold RPMI 1640 culture medium (GIBCO, Grand Island, N.Y.) supplemented with 10% heat-inactivated fetal calf serum (GIBCO). Cells were pelleted by centrifugation at 1500 rpm for 8 minutes. Contaminating red cells were removed by treating the pellet with ammonium chloride lysing buffer (GIBCO) for 2 minutes at 4°C. Cold medium was added and cells were again centrifuged at 1500 rpm for 8 minutes. T lymphocytes were then isolated by separation of the cell suspension on nylon wool columns as follows: Nylon wool columns were prepared by packing approximately 4 grams of washed and dried nylon wool into 20 ml plastic syringes. The columns were sterilized by autoclaving at 250°F for 30 minutes. Nylon wool columns were wetted with warm (37°C) culture medium and rinsed with the same medium. Washed spleen cells resuspended in warm medium were slowly applied to the nylon wool. The columns were then incubated in an upright position at 37°C for 1 hour. Non-adherent T lymphocytes were eluted from the columns with warm culture medium and the cell suspensions were spun as above.

Purified T lymphocytes were resuspended at $10^6$ cells/ml in complete culture medium composed of RPMI 1640 medium with 10% heat-inactivated fetal calf serum, 100 mM glutamine, 1 mM sodium pyruvate, $2 \times 10^{-5}$ M 2-mercaptoethanol and 50 μg/ml gentamycin. Ionomycin was added at 250 ng/ml and PMA at 10 ng/ml. The cell suspension was immediately distributed into 96 well flat-bottom microculture plates (Costar) at 100 μl/well. Control (cyclosporin) medium or various below-indicated dilutions of the sample (above-described methanol extracts) to be tested were then added in triplicate wells at 10 μl/well. The culture plates were then incubated at 37°C in a humidified atmosphere of 5% $CO_2$-95% air for 44 hours. The proliferation of T lymphocytes was assessed by measurement of tritiated thymidine incorporation. After 44 hours of culturing, the cells were pulse-labelled with 2 μCi/well of tritiated thymidine (NEN, Cambridge, MA). After another 4 hours of incubation, cultures were harvested on glass fiber filters using a multiple sample harvester. Radioactivity of filter discs corresponding to individual wells was measured by standard liquid scintillation counting methods (Betacounter). Mean counts per minute of replicate wells were calculated and the results expressed as percent inhibition of tritiated thymidine uptake (proliferation) as follows:

$$\% \text{ Inhibition} = 100 - \left[ \frac{\text{Mean cpm sample tested}}{\text{Mean cpm control medium}} \times 100 \right].$$

The results of % inhibition at various dilutions of the broth samples are presented in the following Table I:

TABLE I

| Sample dilution (WBE) | Broth Sample % Inhibition | | |
|---|---|---|---|
| | RP21 | FKSH | KO (dilution x 5) |
| 1:2,200 | 98 | 98 | 98 |
| 1:4,400 | 92 | 98 | 95 |
| 1:8,800 | 63 | 86 | 83 |
| 1:17,600 | 0 | 55 | 60 |
| 1:35,200 | | 0 | 0 |
| 1:70,400 | | 0 | 0 |

Notes: 1. Mouse T cell cultures were pulsed with $^3$H-thymidine for 4 hours prior to harvesting at 48 hours.

2. Control Cyclosporin A (100 ng/ml) gave 97% inhibition.

3. KQ dilutions were 5 times greater than for RP21 and FKSH.

## EXAMPLE 3

### HPLC Behavior

Conditions:
Solvent composition: Knauer 64 pump delivering acetonitrile:water (13:7) at 1.0 ml/min.
Column: Lichrosorb RP-18 (EM Science) 4.0 x 250 mm, 5 micron irregular particles, no guard column, operated at 40.0°C.
Detector: Knauer type 87 variable wavelength UV detector operating at 205 nanometers, 10 mm pathlength, 1.0 volt per AU. Data recorded in a Nelson Analytical Model 3000 data station, 25 millivolts/full scale.
Injection of 25 microliters of a methanol extract of the RP21 fermentation broth produced a chromatogram containing a broad peak for immunomycin with a retention time of 7.66 minutes. By comparison, an FK-506 macrolide immunosuppressant-containing broth extract afforded a peak with a retention time of 7.82 minutes. The difference in retention times of 9.6 seconds indicated that the two immunosuppressants were not identical. The observed peak in the chromatogram of the immunomycin extract was broad and asymmetrical.

## EXAMPLE 4

### HPLC - Large Scale Separation
To allow confirmation of the biological activity of the HPLC peak at 7.66 minutes, and to obtain some preliminary spectral data, a second methanol extract, being a combination of RP21 and FKSH fermentation broth extracts equivalent to 20 milliliters of whole broth, was used. This extract was concentrated to dryness in vacuo then partitioned between 5 ml of distilled water and 3x5 ml of ethyl acetate. The ethyl acetate fraction was evaporated to dryness and the residue partitioned between 5 ml of 95% methanol and 3x5 ml n-hexane. The resulting defatted methanol extract was evaporated to dryness, reconstituted in 250 microliters of methanol and subjected to preparative HPLC under the following conditions:

Conditions:
Solvent composition: Knauer 64 pump delivering acetonitrile:water (13:7) at 4.6 ml/min.
Column: Lichrosorb RP-18 (EM Science), 10.0 x 250 mm, 7 micron irregular particles, no guard column, operated at ambient temperature (about 20°C).
Detector: Knauer type 87 variable wavelength UV detector operating at 205 nanometers, 0.4 mm pathlength, 1.0 volt/AU. Data were recorded with a Nelson Analytical model 3000 data station, 200 millivolts/full scale.
Fractions: 30 fractions, 4.6 milliliters each.
The entire sample was fractionated in a single experiment. Fractions were grouped in three's for assay, resulting in samples A through J tabulated below in Table II. Portions of each fraction, as well as the hexane extract and aqueous extract from the solvent partition experiment, equivalent to 1 milliliter of whole broth, were sent for T-cell proliferation studies and for antifungal screening. Biological activity data are summarized in the following table. The T-cell proliferation assay was conducted by the procedure described above in Example 2, on a sample methanol broth dilution of 1:30,000.

TABLE II

| Fraction | Antifungal Activity | T-cell Proliferation Assay (1/30,000) % Inhibition |
|---|---|---|
| Aqueous | - | 0 |
| A | - | 24 |
| B | - | 2 |
| C | + | 17 |
| D | + | 88 |
| E | + + | 100 |
| F | + | 3 |
| G | - | 7 |
| H | - | 3 |
| I | - | 14 |
| J | - | 1 |
| Hexane | (trace) | 17 |

As seen, the immunosuppressant and antifungal activities are maximized in fraction E.

## EXAMPLE 5

### Mass Spectroscopy

A sample of the above chromatographic fraction "E" was evaporated to dryness in vacuo and the resulting residues were analyzed by mass spectral analysis.

The following high resolution MS data was obtained on a Finnigan MAT212 instrument in the electron impact mode using the peak matching method with PFK (perfluorokerosene) as the internal standard.

| Assignment | Empirical formula | Calculated value | Found value |
|---|---|---|---|
| M+ | $C_{43}H_{69}NO_{12}$ | 791.4820 | 791.4784 |
| M+ - $H_2O$ | $C_{43}H_{67}NO_{11}$ | 773.4714 | 773.4697 |

As indicated, the assigned empirical formulae are consistent with the obtained high resolution mass values.

## EXAMPLE 6

### Antifungal Assays

Antifungal activity of fermentation broths and HPLC fractions was determined by disc-diffusion susceptibility methods against a panel to generate a spectrum of activity against a panel of 22 filamentous fungi, 3 bacteria and 24 yeasts. The resultant spectrum was compared by computer in a pair-wise fashion to the spectra of previously tested samples and the similarity between pairs was estimated. Samples were also characterized by thin-layer chromatography in nine solvent systems. Mobility of antifungal substances was detected by bioautographic methods. The resultant composite pattern or "spectrum" was compared by computer in a pair-wise fashion with the patterns of previously tested antifungals.

### Crude Broth Extract Assay

Classification studies of a (1:1 WBE) methanol extract of the FKSH fermentation broth revealed that the extract contained at least three antifungal substances by TLC/bioautography. Pattern matching of the antifungal spectrum failed to reveal a similarity with previously tested samples.

HPLC Fractions Assay

The results of studies on the above-described HPLC fractions (C, D, E, and F) revealed that they contained a single antifungal component by TLC/bioautography. All four samples exhibited a similar mobility pattern, clustered with the major antifungal components of the FKSH fermentation broth sample described above. Activity was limited to filamentous fungi with a trace of activity against three yeast strains.

Conclusion

Based upon the results of these experiments, it was concluded that the antifungal spectrum of HPLC fractions B, C, D and E was also consistent with the published description of ascomycin.

**Claims**

1. A process for producing an immunosuppressant, identified as "immunomycin", comprising the step of culturing a strain of <u>Streptomyces hygroscopicus var. ascomyceticus</u> under submerged aerobic fermentation conditions in an aqueous carbohydrate medium containing a nitrogen nutrient at a pH below 8.0 for a sufficient time to produce "immunomycin".

2. The process of Claim 1 wherein said microorganism is ATCC 14891.

3. The process of Claim 1 wherein said medium is "RP21" fermentation medium comprising glucose, glycerol, $(NH_4)_2SO_4$, $KH_2PO_4$, $K_2HPO_4$, L-lysine, nutrisoy and morpholinoethanesulfonic acid (MES) buffer.

4. The process of Claim 1 wherein said medium is "KQ" fermentation medium comprising figs, dextrin, primary yeast, $CoCl_2.6H_2O$ and an antifoaming agent.

5. The process of Claim 1 wherein said medium is "FKSH" fermentation medium comprising glucose, corn steep liquor, primary yeast, corn gluten meal, wheat germ and $CaCO_3$.

6. The broth produced by the process of Claim 1, containing "immunomycin", said broth exhibiting a positive inhibition of T-cell activation by the T-cell proliferation assay.

7. The immunosuppressant product produced by the above-described process of Claim 1.

8. An immunosuppressant, "immunomycin", which exhibits: positive inhibition of T-cell activation by the T-cell proliferation assay and a molecular weight as obtained by mass spectroscopy of 791.4784.

9. A pharmaceutical composition containing a therapeutically effective amount of "immunomycin" in combination with a pharmaceutically acceptable, substantially non-toxic carrier or excipient.

10. The use of "immunomycin" for the preparation of a medicament useful for treating a human host to prevent transplantation rejection, or for treating autoimmune disease or infectious disease.

11. The use of "ascomycin" for the preparation of a medicament useful for treating a human host to prevent transplantation rejection, or for treating autoimmune disease or infectious disease.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | EP-A-0 184 162 (FUJISAWA)<br>* Pages 1-14,27-38,48-59,100-110 *<br>--- | 1,2,9 | C 12 P 1/06<br>A 61 K 35/66<br>A 61 K 39/00 //<br>(C 12 P 1/06<br>C 12 R 1:55 ) |
| D,A | US-A-3 244 592 (TADASHI ARAI)<br>* Whole document *<br>----- | 1,2,9 | |
| | | | TECHNICAL FIELDS SEARCHED (Int. Cl.4)<br><br>C 12 P 1/00<br>A 61 K 35/00<br>A 61 K 39/00<br>C 12 R 1/00 |

The present search report has been drawn up for all claims

| Place of search | Date of completion of the search | Examiner |
|---|---|---|
| THE HAGUE | 14-04-1989 | RAJIC M. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P0401)